# EUROPEAN PATENT SPECIFICATION

(11) **EP 2 256 173 B1**
(45) Date of publication and mention of the grant of the patent: **05.08.2015**
(21) Application number: 09161427.1
(22) Date of filing: 28.05.2009
(51) Int. Cl.: A61F 13/15, A61F 13/551, B32B 27/08, B32B 27/28, B32B 27/32, C09J 7/02

(54) **Release sheet material**
Trennfolienmaterial
Matériau de feuille de décollement

(43) Date of publication of application: 01.12.2010
(73) Proprietor: The Procter & Gamble Company, Cincinnati, OH 45202 (US)
(72) Inventor: Toro, Carlo, 65012 Cepagatti (Pescara) (IT); Salone, Fiorello, 65124 Pescara (IT); Pompei, Enzo, 65129 Pescara (IT)
(74) Representative: Briatore, Andrea

(56) References cited:
- EP-A1- 1 354 575
- WO-A1-96/34029

## Description

### FIELD OF THE INVENTION

The present invention relates to improved release sheet materials for use in connection with disposable absorbent articles, for example sanitary napkins which are typically individually packaged prior to use.

### BACKGROUND OF THE INVENTION

Disposable absorbent articles of personal hygiene are known in the art. Typical examples include sanitary napkins, panty liners, adult incontinence articles, infant diapers, paper towels, bath tissue and facial tissue. Such articles are often used to absorb and retain bodily fluids and other exudates excreted by the human body. Many disposable absorbent articles have the same basic structure: an absorbent core encased between a liquid permeable, user contacting topsheet, which permits liquid to penetrate its thickness and contact the absorbent core where liquid is retained, and a backsheet, which may be liquid impermeable.

While there are a great many variations in the specific structural features of disposable absorbent articles, they are typically presented to consumers in the same manner. Essentially, the disposable absorbent article, irrespective of the specific structural features used, is packaged in a box or bag from which the consumer withdraws the article, as needed. In order to protect the article from soiling or contamination from the time it is removed from the box or bag until the article is used, for example if a woman wanted to carry a sanitary napkin with her for use away from home, the articles may be individually packaged within the box or bag by means of a sheet of material which is wrapped around the individual article.

A typical individual package for disposable absorbent articles is disclosed, for example, in US patent 4,556,146, which describes a disposable absorbent article, typically a sanitary napkin, associated with a wrapper which overlays one major surface of the sanitary napkin. The wrapper extends beyond the perimeter of the disposable absorbent article so that when the disposable absorbent article and the wrapper are folded as a unit, the longitudinal side flaps of the wrapper, which extend beyond the longitudinal sides of the article, may be frangibly sealed thereby providing the disposable absorbent article with an individual package. It is also common to provide the disposable absorbent article with an adhesive element on the garment facing side of the backsheet which, in use, serves to affix the absorbent article to the wearers undergarment thereby maintaining the absorbent article in place against the wearers body. The adhesive element may take the form of a coating of adhesive which is in strips or other suitable pattern. For example, the garment facing side of the backsheet can be coated uniformly with a layer of pressure sensitive hot melt adhesive. The wrapper overlays the garment facing side of the backsheet with the longitudinal flap portions extending beyond the longitudinal perimeter segments of the absorbent article. The wrapper typically is not folded onto or otherwise brought into contact with the body facing side of the topsheet; in other words, the surface of the wrapper facing the garment facing side of the backsheet is in face to face relation substantially with said side only of the backsheet. The wrapper is typically releasably affixed to the disposable absorbent article, e.g. a sanitary napkin, by the aforementioned adhesive element. When an adhesive element is used in this manner, it is not necessary to provide the absorbent articles with a separate release sheet in order to protect the adhesive element before use, as this function is provided by the wrapper.

To individually package the absorbent article, the article and the affixed wrapper can be typically folded as a unit. That is, they are folded together with the wrapper remaining in place with respect to the absorbent article. Typically, the absorbent article is folded lengthwise into thirds about two fold axes. The longitudinal side flaps or flap portions of the wrapper are frangibly sealed using any of the well known sealing techniques. For example, the longitudinal flap portions may be heat sealed, glued, ultrasonically bonded, or crimped.

In use, the individually packaged absorbent article is provided to a user. The user may then break the frangible seals, unfold the wrapper/absorbent article unit and separate the wrapper from the absorbent article, for example a sanitary napkin, exposing the adhesive element. The absorbent article may then be used as such devices normally are, typically being adhered by means of the adhesive element to the crotch portion of an undergarment, which is subsequently worn.

An advantage of an individually packaged disposable absorbent article, for example a sanitary napkin or a pantiliner, is discreetness, as a user does not need to take with her an entire box or bag of products, but only a small number of individually packaged articles, as needed for subsequent use, for example when staying away from home. In a high speed production line the material of the wrapper can be typically provided in a continuous web; the adhesive element may be provided in selected areas directly onto the wrapper material continuous web, and then absorbent articles are provided onto each respective adhesive element, by adhering thereon the garment facing side of the backsheet. The wrapper material continuous web may be folded together with the applied absorbent article, and cut and sealed in order to form individually packaged articles. When the user, after unfolding the package and breaking the seals, separates the absorbent article from the wrapper material, at least some of the adhesive element, and typically substantially the entire adhesive element, remains on the garment facing surface of the backsheet since the respective surface of the wrapper element is typically provided with means having an inferior adhesion to the adhesive material.

Typically the wrapper material, e.g. a polyolefin film, can include a silicone composition which provides the wrapper material with the needed inferior adhesion to the adhesive material, i.e., namely with its adhesive release property. The silicone composition can be typically provided to the already formed sheet material as a coating, for example a known coating technique comprises applying to the sheet material used for the wrapper a layer of a silicone precursor composition, for example comprising a crosslinkable organosilicon prepolymer, which is then cured, namely crosslinked, and anchored to the sheet material surface. Curing/crosslinking can be typically performed by providing energy to the precursor composition in presence of a suitable catalyst or initiator, for example as UV radiation in the presence of a photoinitiator, or heat in the presence of a catalyst.

Providing the silicone composition to the wrapper material as a coating constitutes a multi step process, involving the formation, or in any case the provision, of a finished wrapper material which has to be then coated with the silicone precursor composition, followed by the curing/crosslinking step. Also, this process is energy consuming, as curing/crosslinking is achieved by means of energy.

Alternative processes for the production of wrapper materials, e.g. typically polyolefin films, already comprising a release, e.g. a silicone, material, have been developed in order to cornprise a single manufacturing step, i.e. a film formation process, typically by extrusion, also including the provision of the release material.

For example, as described in European application EP 1354575, a wrapper material for feminine hygiene pads comprises a so called functional layer obtainable by extrusion of a siloxane polymer masterbatch mixed with a thermoplastic resin, e.g. polyethylene. The functional layer provides the adhesive release property, and has to be somehow associated to other polyolefin substrate layers in order to form a quite complex multilayer structure.

Said composite, multilayer structures, however, are not satisfactory in terms of stability. In use, in fact, or even in the manufacturing process to form the wrapped articles, the layer or layers comprising the silicone based material tends to delaminate from the adjacent polyolefin substrate material. In addition, the silicone based polymer tends to at least partially migrate e.g. into the adjacent substrate layer or layers, and ultimately into the adhesive material of the absorbent article contracting it in the wrapped configuration. This can spoil the adhesive properties of the adhesive material, which can hence show a lower adhesion capacity towards the fabric material of the undergarment.

An alternative approach could be to form a multilayer film structure for a wrapper material e.g. by means of co-extrusion, i.e. by extruding substantially at the same time two or more layers comprising different materials, for example respectively a silicone based material and a polyolefin such as a polysiloxane and polyethylene respectively, in order to form the composite layered film structure typically comprising a structural layer and a release layer. Possibly, also, an intermediate layer comprising a so called "adhesion promoter"material can be co-extruded together with the silicone based material and the polyolefin material in an attempt to improve the combination of the structural layer with the release layer, particularly in terms of mechanical stability and resistance to further manufacturing steps, and also to aging.

WO 96/34029 describes a co-extruded three layers release sheet containing a polydiorganosiloxane polymer external layer.

However, these techniques can be less than fully successful in solving the problem of mechanical stability of the composite layered film structure on the one hand, and also of the migration of the silicone based material on the other hand. Common drawbacks can typically include delamination of composite films upon minimal stress, e.g. during further manufacturing steps in order to prepare wrapped absorbent articles, or also in use, e.g. when detaching an absorbent article from the wrapper material. Also, the silicone based material may still tend to migrate into the other layer or layers, thus possibly providing an unpleasant "oily" feel to the film structure, and could even diffuse into the adhesive material upon contact with it typically in the packaged article during storage.

It is therefore desirable to provide a flexible release sheet material for use as wrapper material for disposable absorbent articles, which comprises a multilayer structure obtainable by means of co-extrusion, and which is also more stable, i.e. less prone to the risk of delamination upon subsequent processing and use, and also comprising a release material, e.g. typically a silicone based material, which has a reduced tendency to migrate into adjacent layers and/or in the adhesive material of the absorbent article which is in contact thereto in the packaged configuration.

### SUMMARY OF THE INVENTION

The present invention addresses the above need by providing a flexible release sheet material comprising a co-extruded multilayer film including a first layer comprising a polyolefin, and a second layer comprising a silicone based material selected from polydimethylsiloxane-urea (PDMS-urea) copolymers, and from 10% to 50% by weight, or from 10% to 40% by weight, or from 15% to 35% by weight, or also from 20% to 25% by weight, of a compatibilizer compound selected from ethylene - acrylic ester - maleic anhydride terpolymers, ethylene acrylic acid copolymers, and ethylene vinyl acetate copolymers.

### BRIEF DESCRIPTION OF THE DRAWINGS

Figure 1 is a partially cut away perspective view of an absorbent article and its associated wrapper prior to being folded and sealed.
Figure 2 is a partially cut away perspective view of an absorbent article and its associated wrapper after they have been folded and sealed to form an individually packaged absorbent article.
Figure 3 is cross section of a flexible release sheet material according to an embodiment of the present invention.
Figure 4 is a cross section of a flexible release sheet material according to an alternative embodiment of the present invention.
Figure 5 is a schematic representation of an apparatus for the manufacturing of a flexible release sheet material according to the present invention.

### DETAILED DESCRIPTION OF THE INVENTION

Referring now to the drawings there is shown an individually packaged disposable absorbent article embodying the teachings of the present invention. As used herein the term "absorbent article" refers to those articles intended to absorb and retain liquid and in particular to those articles which are placed against or in proximity to a wearer's body to absorb and contain the various liquids discharged from the body (e.g. blood, menses, urine). A "disposable absorbent article" is an absorbent article which is intended to be discarded after a single use (i.e., they are not intended to be laundered or otherwise restored and reused). Typical disposable absorbent articles according to the present invention can be absorbent articles for feminine hygiene such as sanitary napkins and panty liners, commonly referred to collectively as catamenial pads, or light incontinence product.

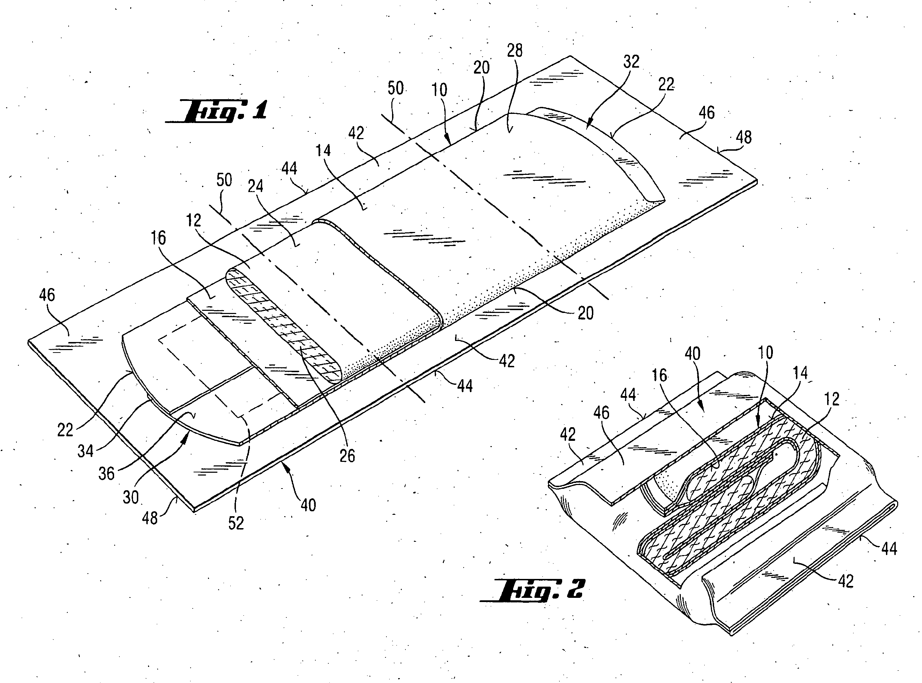

FIG. 1 is a partially cut away perspective view of a catamenial pad 10 and a wrapper 40 prior to being folded and sealed as set forth in greater detail herein below. A catamenial pad is a disposable absorbent article which is worn by females external to and in the proximity of the urogenital region and which is intended to absorb and contain menstrual fluids and other vaginal discharges. As used herein the term "catamenial pad" includes pantiliners which are often worn by females external to the urogenital region between periods of heavy menstrual flow and which are intended to absorb light menstrual flow and nonmenstrual vaginal discharges. The primary difference between catamenial pads used during periods of heavy menstrual flow and catamenial pads used between periods of heavy menstrual flow (i.e., pantiliners) is the absorbent capacity of the pad.

While the present invention will be described with reference to a catamenial pad, it should be understood that the present invention has application in the context of other disposable absorbent articles such as, for example, light incontinence products. Further, the teachings of this invention have application to catamenial pads manufactured according to the teachings of any of the multitudinous references in the catamenial pad art. A typical catamenial pad embodiment will now be described briefly.

As can be seen in FIG. 1, the catamenial pad 10 basically can comprise an absorbent pad 12, an envelope sheet 14 and a barrier 16. The catamenial pad 10 has a perimeter generally comprising longitudinal perimeter segments, or longitudinal sides 20 and transverse perimeter segments, or transverse ends 22. The perimeter defines the outer boundary of the catamenial pad 10, while the longitudinal perimeter segments 20 and the transverse perimeter segments 22 define the outer boundary of the catamenial pad 10 along each longitudinal side and each transverse end respectively.

The absorbent pad 12 is generally compressible, conformable, and non-irritating to the user's skin and may be manufactured from a wide variety of absorbent materials which are capable of absorbing and retaining liquids. For example, a batt of absorbent fibres, a multiplicity of plies of creped cellulose wadding, or any equivalent material may be used. The absorbent capacity of the material used, however, should be sufficient to absorb and retain the expected liquid loading in the intended use of the absorbent article without undue bulk. An example of a suitable catamenial pad 10 intended to receive heavy menstrual discharges of approximately 40 millilitres, may include about 8 grams of comminuted wood pulp, generally referred to as airfelt.

The shape and dimensions of the absorbent pad 12 can be selected to permit the disposable absorbent article to conform to and fit about the portion of the body against which it will be placed. Often, as in the embodiment illustrated, the general shape and dimensions of the catamenial pad 10 can be determined by the shape and dimensions of the absorbent pad 12. In the embodiment illustrated in the figures, the shape and dimensions of the absorbent pad 12 were selected to permit the catamenial pad 10 to conform to the urogenital region of the wearer's body. While the shape and dimensions of the absorbent pad 12 may be varied, it has been found that a generally planar configuration having a first major surface, or body facing side, 24 and a second major surface, or garment facing side, 26 can be suitable. The first major surface 24 is that surface of the absorbent pad 12 facing toward the source of liquid (i.e. toward the wearer's body) and the second major surface 26 is that surface of the absorbent pad 12 facing away from the source of liquid. An example of a suitable catamenial pad 10 can have a generally rectangular, planar shaped absorbent pad 12 having a length of about 9.0 inches (22.9 centimetres) and a width of about 2.5 inches (6.4 centimetres). It should be understood, however, that other shapes (e.g. elongated ovals, triangles, squares, etc.) and other dimensions may be used.

The catamenial pad 10 may include an envelope sheet 14 that encases the absorbent pad 12 and is preferably compliant, soft feeling, and non-irritating to the wearer's body. The envelope sheet 14 can help maintain the structural integrity of the absorbent pad 12 and has a first and a second end flap 30 and 32 respectively. The envelope sheet 14 may be wrapped about the absorbent pad 12 and may be affixed to itself along a seam 34 which is adjacent the second major surface 26 and which traverses the catamenial pad 10 longitudinally. The first and second flaps 30 and 32 respectively extend beyond the transverse ends of the absorbent pad 12 and are typically sealed so as to completely encase the absorbent pad 12 within the envelope sheet 14. The portion of the envelope sheet 14 overlaying the first major surface 24 is the topsheet portion 28 corresponding to the body facing side of the article, and the portion of the envelope sheet 14 overlaying the second major surface 26 is the backsheet portion 36 of the envelope sheet 14, corresponding to the garment facing side of the article. The topsheet portion 28 is liquid permeable. In use the topsheet portion 28 may contact the skin of the catamenial pad wearer and permit the transmission of liquid through its thickness to the absorbent pad 12 where the liquid may be retained.

There are many suitable materials from which the envelope sheet 14 may be manufactured. The topsheet portion 28 may be manufactured from either hydrophobic or hydrophilic fibres and may, for example, be carded, spun bonded, melt blown, or air laid. Alternatively, the topsheet portion 28 may be a continuous film or sheet of, for example, thermoplastic material which is apertured. A suitable topsheet portion 28 is described in U.S. Pat. No. 4,324,246 which issued to Mullane et al on Apr. 13, 1982.

The topsheet portion 28 and the backsheet portion 36 may either be integral (i.e. the backsheet portion 36 and the topsheet portion 28 are separate elements affixed to each other) or unitary (i.e. the backsheet portion 36 and the topsheet portion 28 are formed from a continuous and undivided sheet of material) and may either have the same or different physical properties. The embodiment of FIG. 1 shows the topsheet portion 28 and the backsheet portion 36 as being unitary.

To help prevent liquids absorbed by the absorbent pad 12 from penetrating through the backsheet portion 36, it may be advantageous to interpose a barrier 16 at the interface between the second major surface 26 of the absorbent pad 12 and the backsheet portion 36. The barrier 16 may be manufactured from any flexible, liquid impermeable material which is non-irritating to the wearer. In certain embodiments, the barrier 16 may be a sheet of polyethylene which is coincident with the backsheet portion 36.

Alternatively, the envelope sheet 14 may comprise a topsheet portion 28 and a backsheet portion 36 which are made integral with each other by affixing them together about their periphery. In such embodiments, the topsheet portion 28 may be at least partially liquid impervious and the backsheet portion 36 may be liquid pervious or wholly or partially liquid impervious.

It is common to provide the catamenial pad 10 with an adhesive element 52, partially shown in dotted line in FIG. 1. The adhesive element 52 is positioned on the garment facing side of the article, namely on the backsheet portion 36 and, in use, serves to affix the catamenial pad 10 to the wearer's undergarments thereby maintaining the catamenial pad 10 in place against the wearer's body. The adhesive element 52 may take the form of a coating of adhesive which is in strips or any other suitable pattern. The backsheet portion 36 may be coated uniformly with a layer of a pressure sensitive hot melt adhesive such as, for example, NS34-2823 as manufactured by National Starch and Chemical of Bridgewater, N.J.

In accordance with the teachings of this invention, a wrapper 40 is associated with, and typically can have dimensions generally larger than those of the catamenial pad 10. Thus, the wrapper 40 has longitudinal flap portions 42 comprising that portion of the wrapper 40 between the longitudinal edge 44 of the wrapper and the longitudinal perimeter segment or longitudinal side 20 of the catamenial pad 10. In the embodiment illustrated in FIG. 1, the wrapper 40 also has transverse flap portions 46 comprising that portion of the wrapper 40 between the transverse edges 48 of the wrapper 40 and the transverse perimeter segments or transverse ends 22 of the catamenial pad 10.

The wrapper 40 is shown overlaying the garment facing side of the absorbent article, namely the backsheet portion 36, with the longitudinal flap portions 42 typically extending beyond the longitudinal perimeter segments 20. In the absorbent shown in FIG. 1, the wrapper 40 is not folded onto or otherwise brought into contact with the topsheet portion 28. In other words, the surface of the wrapper 40 facing the backsheet portion 36 is in face to face relation with the backsheet portion 36 only. The wrapper 40 is releasably affixed to the catamenial pad 10 by the aforementioned adhesive element 52, hence it is also defined as a releasable wrapper 40 according to the present invention. If an adhesive element is used in this manner, it is not necessary to provide the absorbent article with a separate release sheet as is commonly done in prior art devices, as the function of protecting the adhesive element from contamination prior to use is provided by the wrapper.

To individually package the catamenial pad 10, the catamenial pad 10 and the affixed wrapper 40 can be folded as a unit. That is, they can be folded together with the wrapper 40 remaining in place with respect to the catamenial pad 10. According to the present invention, the absorbent article and the wrapper can be folded as a unit about at least one fold-axis, e.g. a longitudinal or a transverse fold-axis, in order to define a packaged absorbent article. In certain embodiments, the catamenial pad 10 may be folded lengthwise into thirds about two transverse fold-axes 50, as shown in FIG. 2, to define a package comprising the absorbent article. The longitudinal flap portions 42 can be frangibly sealed using any of the well-known sealing techniques. For example, the longitudinal flap portions 42 may be heat sealed, glued, or ultrasonically bonded.

In use, the individually packaged catamenial pad is provided to a user. The user may then break the seals, unfold the catamenial pad 10, and separate the wrapper 40 from the catamenial pad 10. The catamenial pad 10 may then be used as such devices normally are.

Means having a reduced adhesion to the adhesive material of the adhesive element 52 can be typically provided into the material of the wrapper 40, namely on the surface which is meant to contact, and be affixed to, the adhesive element 52 on the garment facing surface of the absorbent article in an individually packaged and wrapped absorbent article as illustrated in Figures 1 and 2.

According to an embodiment of the present invention, a flexible release sheet material is provided, typically for use as a releasable wrapper for an individually packaged absorbent article. The flexible release sheet material comprises a co-extruded multilayer film; the flexible release sheet material according to the present invention is therefore a multilayer film obtainable by means of co-extrusion, i.e. a co-extruded film comprising at least two layers.

Co-extrusion refers to the extrusion of multiple layers of material simultaneously. This type of extrusion can typically utilize two or more extruders to melt and deliver a steady volumetric throughput of different viscous thermoplastic materials to a single extrusion head (die) which will extrude the materials in the desired form, i.e., in the context of the present invention, as a multilayer film. This technology can be used on any of the processes for film forming, such as sheet/film extrusion or blown film extrusion. The respective layer thicknesses can be controlled according to known means, i.e. typically by the relative speeds and sizes of the individual extruders delivering the materials to the single extrusion die. Co-extrusion can have the advantage that a multiple layer film structure can be manufactured in a single and relatively simple process step, with no need of post processing. For example a composite film can be produced having a layer or layers providing mechanical characteristics such as flexibility, resilience, strength, and a layer or layers providing desired surface finishing, such as for example an adhesive release surface.

According to an embodiment of the present invention, a flexible release sheet material comprising a co-extruded multilayer film, i.e. a multilayer film obtainable by means of co-extrusion, is provided which has both mechanical and chemical stability, typically for use in a releasable wrapper for an individually packaged absorbent article. A releasable wrapper made of this release sheet material, and an absorbent article individually packaged in a releasable wrapper made of this flexible release sheet material are also within the scope of the present invention. The flexible release sheet material can be obtained by means of a careful selection of the components of the different layers.

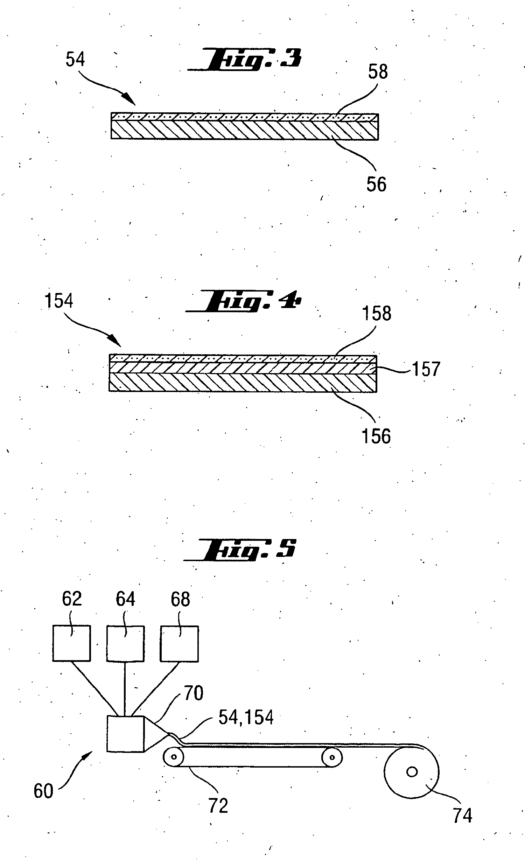

As schematically illustrated in Figure 3, the flexible release sheet material according to an embodiment of the present invention comprises the co-extruded multilayer film 54, which can typically include at least two layers, respectively a first layer 56 comprising a polyolefin, and at least a second layer 58 comprising a silicone based material and a so called compatibilizer compound, wherein the silicone based material can be typically a copolymer, selected among polydimethylsiloxane-urea (PDMS-urea) copolymers, while the compatibilizer compound can be typically selected from ethylene - acrylic ester - maleic anhydride terpolymers, ethylene acrylic acid copolymers, ethylene vinyl acetate copolymers. The compatibilizer compound can typically constitute from 10% to 50% by weight, or from 10% to 40% by weight, or from 15% to 35% by weight, or also from 20% to 25% by weight, of the second layer 58 also comprising the silicone based material selected as described above.

The polyolefin comprised in the first layer 56 of the multilayer film 54 can typically be polyethylene, such as for example linear low density polyethylene (LLDPE). Other olefin polymers can also be selected, such as for example polypropylene, for the first layer 56.

Without being bound to any theory, it is believed the selection of the silicone based polymer in the second layer 58 among copolymers of a polydimethylsiloxane and urea, and the combination in the same second layer 58 with a compatibilizer compound selected from ethylene - acrylic ester - maleic anhydride terpolymers, ethylene acrylic acid copolymers, and ethylene vinyl acetate copolymers, as described above, can provide in the co-extrusion process where the second layer 58 is co-extruded together with the first layer 56 comprising a polyolefin, for example polyethylene, in order to form the multilayer film 54, for a rather oriented internal structure. Namely, the less polar portions of the copolymer, typically the PDMS blocks, can be preferentially oriented towards the film side opposite to that of the first layer 56, i.e. towards the outer surface of the multilayer film 54, which in use as a release sheet material, for example as a releasable wrapper 40, are meant to get in contact with the adhesive element 52 of the catamenial pad 10, and provide the release properties. At the same time, the substantially oriented second layer 58 in the multilayer film 54 can also provide a more stable bonding to the first layer 56 comprising the polyolefin. Overall, the multilayer film of the present invention can provide a better mechanical stability, where the at least two layers 56 and 58 are more effectively bonded together in a robust structure, capable of withstanding the stresses of further manufacturing steps and of subsequent use. At the same time the structure can be also considered to be more chemically stable, in that the molecular orientation of the different blocks in the copolymers can tend to remain over time, and therefore the polymers, namely the PDMS copolymer and the compatibilizer copolymer, can have a reduced tendency to migrate with ageing, possibly owing to intermolecular interaction forces, i.e. typically hydrogen bonding, thus particularly avoiding migration into the adhesive upon prolonged contact during storage and before use of the absorbent article.

According to an alternative embodiment of the present invention, the first layer 56 of the multilayer film 54 comprised in the flexible release sheet material can further comprise, in addition to the polyolefin, a compatibilizer compound selected from the same components described above for the second layer 58. The amount of the compatibilizer compound in the first layer 56 can be substantially the same as for the second layer 58, that is the compatibilizer compound can typically constitute from 10% to 50% by weight, or from 10% to 40% by weight, or from 15% to 35% by weight, or also from 20% to 25% by weight, of the first layer 56 also comprising the polyolefin as described above. It is believed this can further improve the bonding between the first and the second layer 56 and 58 in a flexible release sheet material 54 similar to that illustrated in Figure 3. In certain embodiments of the present invention, the amount of the compatibilizer compound in the first layer 56 can even go up to 70% by weight, or also to 80% by weight.

According to a further alternative embodiment of the present invention, a flexible release sheet material can comprise a co-extruded multilayer film 154 as illustrated in Figure 4, further comprising a third layer 157 between a first layer 156, comprising the polyolefin, and a second layer 158, comprising the silicone based material and the compatibilizer compound. The third layer 157 can be typically constituted of the compatibilizer compound, typically the same material as that comprised in the second layer 158. An intermediate third layer 157 constituted by the compatibilizer compound, and interposed between the first and the second layer 156, 158, can further improve the bonding between the first layer 156 comprising the polyolefin, and as explained typically providing the multilayer release sheet material 154 with the desired mechanical properties, and the second layer, which instead can be typically meant to provide the release properties.

According to an embodiment of the present invention the flexible release sheet material can be actually constituted by a co-extruded multilayer film as described above.

According to an embodiment of the present invention the compatibilizer compound can be for example an ethylene - acrylic ester - maleic anhydride terpolymer. Typically the maleic anhydride content in the terpolymer could be relatively low, for example below 1% by weight, down to about 0.3% by weight, while the acrylic ester content can be from 10% to 30% by weight. The compatibilizer compound can be selected among the ethylene - acrylic ester - maleic anhydride terpolymers sold by Arkema under the trade name Lotader^{®} MAH, such as for example Lotader^{®} 4403.

According to another embodiment of the present invention the compatibilizer compound can be an ethylene -acrylic acid copolymer. Typically the content of acrylic acid can be for example up to 10% by weight. An example can be the compound sold as Primacor^{®} 3460 by Dow Chamical, from the Primacor^{®} family.

As for the silicone based material, according to an embodiment of the present invention it can be a PDMS-urea copolymer, such as for example the materials sold by Wacker Chemie AG under the Geniomer^{®} trade name, such as e.g. Geniomer^{®} 80.

According to an embodiment of the present invention, the thickness of the first layer 56 can be from 5 µm to 60 µm, or from15 µm to 40 µm, or also from 20 µm to 30 µm.

The thickness of the second layer 58 can be from 0.2 µm to 5 µm, or from 0.5 µm to 2 µm

The thickness of the third layer, when present, can be from 1 µm to 10 µm, or from 4 µm to 8 µm

The overall thickness of the multilayer film constituting the flexible release sheet material according to an embodiment of the present invention can be from 8 µm to80 µm, or from 10 µm to 40 µm, or from 15 µm to 30 µm.

A process and an apparatus for making a flexible release sheet material according to the present invention is schematically illustrated, in a simplified way, in Figure 5. An apparatus 60 for co-extrusion is fed through reservoirs 62, 64, 66 of the selected polyolefin material, of the selected silicone based material, and of the selected compatibilizer compound, respectively. The different materials can be mixed together as appropriate with known means and provide the selected feeds to the extrusion die 68 in order to provide a multilayer film 54, 154 having the desired structure, i.e. comprising for example two or three layers with specific compositions as explained above. The multilayer film can be moved through e.g. a conveyor belt 70 to further manufacturing steps, or wound in a roll 72 for storage and subsequent use.

### Example

A flexible release sheet material according to the invention as Example 1 was prepared by co-extruding a multilayer film having a first layer 22 µm thick of 100% LDPE (Low Density Polyethylene), a second layer 2 µm thick comprising 80% by weight of a PDMS-urea copolymer Geniomer^{®} 80 sold by Wacker Chemie AG and 20% by weight of a compatibilizer compound sold by Arkema as Lotader^{®} 4403, and a third layer 6 µm thick between the first and the second layer, of 100% Lotader^{®} 4403.

As a comparative example, a sheet material was prepared by co-extruding a two layer film having a first layer 22 µm thick of 100% LDPE and a second layer 2 µm thick of 100% Lotader^{®} 4403.

As a reference, a commercial release sheet material sold by Nordenia under the code NOR^{®} Pouch RPW PE NDG V5was also provided, consisting of a LDPE film which has received a UV cured silicone coating according to the known technology.

The release force of the three sheet materials was tested according to the FINAT Test Method no. 10 using a Tesa Tape 7475, which can be considered representative of the adhesives commonly used to affix absorbent articles to the wearer's undergarment during wear. The results in N/25.4 mm are summarized in the table below:

| | Comparative example | Example 1 | Reference (Nordenia NOR^{®} Pouch) |
|---|---|---|---|
| Second layer (thickness) | 100% Geniomer^{®} 80 2 µm | 80% Geniomer^{®} 80 20% Loader^{®} 4403 2 µm | UV silicone coating |
| Third layer (thickness) | None | 100% Lotader^{®} 4403 6 µm | None |
| First layer (thickness) | 100% LDPE 22 µm | 100% LDPE 22 µm | 100% LDPE |
| Release force (N/25.4 mm) | Delamination Not measurable | 2.63 | 0.2 |

The results in the table show that the comparative example does not have any release action, as the second layer, meant to provide the release properties, simply delaminates from the first layer of LDPE. Example 1 instead provides a release force which, although higher than that measured for the reference commercial material, is perfectly acceptable for a release sheet material providing a releasable wrapper for an individually packaged absorbent article. Example 1 is a release sheet material made according to a simple and cost effective process in a single manufacturing step.

## Claims

1. A flexible release sheet material comprising a co-extruded multilayer film including a first layer comprising a polyolefin, and a second layer comprising a silicone based material selected from polydimethylsiloxane-urea (PDMS-urea) copolymers, and from 10% to 50% by weight, preferably from 10% to 40% by weight, more preferably from 15% to 35% by weight, most preferably from 20% to 25% by weight, of a compatibilizer compound selected from ethylene - acrylic ester - maleic anhydride terpolymers, ethylene acrylic acid copolymers, and ethylene vinyl acetate copolymers.

2. A flexible release sheet material according to claim 1, wherein said first layer further comprises from 10% to 50% by weight, preferably from 10% to 40% by weight, more preferably from 15% to 35% by weight, most preferably from 20% to 25% by weight, of a compatibilizer compound selected from ethylene acrylic ester maleic anhydride terpolymers, ethylene acrylic acid copolymers, ethylene vinyl acetate copolymers.

3. A flexible release sheet material according to any preceding claim, wherein said multilayer film further comprises a third layer comprised between said at least first and second layers, said third layer constituted of a compatibilizer compound selected from ethylene acrylic ester maleic anhydride terpolymers, ethylene acrylic acid copolymers, ethylene vinyl acetate copolymers.

4. A flexible release sheet material according to any preceding claim, wherein said compatibilizer compound is an ethylene acrylic ester maleic anhydride terpolymer.

5. A flexible release sheet material according to any preceding claim, wherein said polyolefin is polyethylene.

6. A flexible release sheet material according to any preceding claim, wherein said first layer has a thickness of 5 µm to 60 µm, preferably from 15 µm and 40 µm, more preferably from 20 µm and 40 µm.

7. A flexible release sheet material according to any preceding claim, wherein said second layer has a thickness of 0.2 µm to 5 µm, preferably from 0.5 µm and 2 µm.

8. A flexible release sheet material according to claim 3, wherein said third layer has a thickness of 1 µm to 10 µm, preferably from 4 µm and 8 µm.

9. A flexible release sheet material according to any preceding claim, wherein said flexible release sheet material has a thickness of 8 µm to 80 µm, preferably of 10 µm to 40 µm, more preferably of 15 µm to 30 µm.

10. A releasable wrapper for an individually packaged absorbent article, said releasable wrapper comprising a flexible release sheet material according to any preceding claim.

11. An individually packaged absorbent article comprising an absorbent article having a body facing side, a garment facing side, two longitudinal sides and two transverse ends, said absorbent article having an adhesive element on said garment facing side, a releasable wrapper overlaying said garment facing side of said article and releasably affixed to said adhesive element, said absorbent article and said wrapper being folded as a unit about at least one fold-axis to define a package comprising said absorbent article, wherein said releasable wrapper is according to claim 10.

12. A process for making a flexible release sheet material according to any of claims 1 to 9, comprising the steps of:
providing a first feed comprising a polyolefin and, optionally, from 10% to 50% by weight of a compatibilizer compound selected from ethylene - acrylic ester - maleic anhydride terpolymers, ethylene acrylic acid copolymers, ethylene vinyl acetate copolymers,
providing a second feed comprising a silicone based material selected from polydimethylsiloxane-urea copolymers and from 10% to 50% by weight, preferably from 10% and 40% by weight, more preferably from 15% to 35% by weight, most preferably from 20% to 25% by weight, of a compatibilizer compound selected from ethylene - acrylic ester - maleic anhydride terpolymers, ethylene acrylic acid copolymers, ethylene vinyl acetate copolymers,
optionally providing a third feed consisting of a compatibilizer compound selected from ethylene - acrylic ester - maleic anhydride terpolymers, ethylene acrylic acid copolymers, ethylene vinyl acetate copolymers,
co-extruding said first, second and optionally third feed into a multilayer film, wherein said first material provides a first layer, said second material provides a second layer, and said optional third material provides an optional third layer comprised between said first and said second layers.

## Patentansprüche

1. Flexibles Trennlagenmaterial, umfassend eine coextrudierte mehrschichtige Folie, die eine erste Schicht aufweist, die ein Polyolefin umfasst, und eine zweite Schicht, die ein Material auf Silikonbasis umfasst, das ausgewählt ist aus Polydimethylsiloxanharnstoff-Copolymeren (PDMS-Harnstoff-Copolymeren), und zu 10 Gew.-% bis 50 Gew.-%, vorzugsweise zu 10 Gew.-% bis 40 Gew.-%, mehr bevorzugt zu 15 Gew.-% bis 35 Gew.-%, am meisten bevorzugt zu 20 Gew.-% bis 25 Gew.-% eine Vermittlerverbindung, ausgewählt aus Ethylenacrylester-Maleinsäureanhydrid-Terpolymeren, Ethylenacrylsäure-Copolymeren und Ethylenvinylacetat-Copolymeren.

2. Flexibles Trennlagenmaterial nach Anspruch 1, wobei die erste Schicht ferner zu 10 Gew.-% bis 50 Gew.-%, vorzugsweise zu 10 Gew.-% bis 40 Gew.-%, mehr bevorzugt zu 15 Gew.-% bis 35 Gew.-%, am meisten bevorzugt zu 20 Gew.-% bis 25 Gew.-% eine Vermittlerverbindung umfasst, die ausgewählt ist aus Ethylenacrylester-Maleinsäureanhydrid-Terpolymeren, Ethylenacrylsäure-Copolymeren, Ethylenvinylacetat-Copolymeren.

3. Flexibles Trennlagenmaterial nach einem der vorstehenden Ansprüche, wobei die Mehrschichtfolie ferner eine dritte Schicht umfasst, die zwischen wenigstens einer ersten und einer zweiten Schicht angeordnet ist, wobei die dritte Schicht aus einer Vermittlerverbindung gebildet ist, die ausgewählt ist aus Ethylenacrylester-Maleinsäureanhydrid-Terpolymeren, Ethylenacrylsäure-Copolymeren, Ethylenvinylacetat-Copolymeren.

4. Flexibles Trermlagenmaterial nach einem der vorstehenden Ansprüche, wobei die Vermittlerverbindung ein Ethylenacrylester-Maleinsäureanhydrid-Terpolymer ist.

5. Flexibles Trennlagenmaterial nach einem der vorstehenden Ansprüche, wobei das Polyolefin Polyethylen ist.

6. Flexibles Trennlagenmaterial nach einem der vorstehenden Ansprüche, wobei die erste Schicht eine Dicke von 5 µm bis 60 µm, vorzugsweise von 15 µm und 40 µm, mehr bevorzugt von 20 µm und 40 µm aufweist.

7. Flexibles Trennlagenmaterial nach einem der vorstehenden Ansprüche, wobei die zweite Schicht eine Dicke von 0,2 µm bis 5 µm, vorzugsweise von 0,5 µm und 2 µm aufweist.

8. Flexibles Trennlagenmaterial nach Anspruch 3, wobei die dritte Schicht eine Dicke von 1 µm bis 10 µm, vorzugsweise von 4 µm und 8 µm aufweist.

9. Flexibles Trennlagenmaterial nach einem der vorstehenden Ansprüche, wobei das flexible Trennlagenmaterial eine Dicke von 8 µm bis 80 µm, vorzugsweise von 10 µm bis 40 µm, mehr bevorzugt von 15 µm bis 30 µm aufweist.

10. Lösbare Umhüllung für einen einzeln verpackten Absorptionsartikel, wobei die lösbare Umhüllung ein flexibles Trennlagenmaterial nach einem der vorstehenden Ansprüche umfasst.

11. Einzeln abgepackter Absorptionsartikel, der einen Absorptionsartikel mit einer körperseitigen Seite, einer bekleidungsseitigen Seite, zwei Längsseiten und zwei Querenden umfasst, wobei der Absorptionsartikel ein Klebeelement auf der bekleidungsseitigen Seite umfasst, wobei eine lösbare Umhüllung auf der bekleidungsseitigen Seite des Artikels aufliegt und lösbar an dem Klebeelement befestigt ist, wobei der Absorptionsartikel und die Umhüllung als eine Einheit um mindestens eine Faltachse gefaltet sind, um ein Päckchen zu bilden, das den Absorptionsartikel umfasst, wobei die lösbare Umhüllung Anspruch 8 entspricht.

12. Verfahren zum Herstellen eines flexiblen Trennlagenmaterials nach einem der Ansprüche 1 bis 9, das die Schritte umfasst:
Bereitstellen eines ersten Beschickungsmaterials, umfassend ein Polyolefin und wahlweise zu 10 Gew.-% bis 50 Gew.-% einer Vermittlerverbindung, die ausgewählt ist aus Ethylenacrylester-Maleinsäureanhydrid-Terpolymeren, Ethylenacrylsäure-Copolymeren, Ethylenvinylacetat-Copolymeren,
Bereitstellen eines zweiten Beschickungsmaterials, umfassend ein Material auf Silikonbasis, das ausgewählt ist aus Polydimethylsiloxanharnstoff-Copolymeren, und zu 10 Gew.-% bis 50 Gew.-%, vorzugsweise zu 10 Gew.-% und 40 Gew.-%, mehr bevorzugt zu 15 Gew.-% bis 35 Gew.-%, am meisten bevorzugt zu 20 Gew.-% bis 25 Gew.-% eine Vermittlerverbindung, die ausgewählt ist aus Ethylenacrylester-Maleinsäureanhydrid-Terpolymeren, Ethylenacrylsäure-Copolymeren, Ethylenvinylacetat-Copolymeren,
optionales Bereitstellen eines dritten Beschickungsmaterials, umfassend eine Vermittlerverbindung, die ausgewählt ist aus Ethylenacrylester-Maleinsäureanhydrid-Terpolymeren, Ethylenacrylsäure-Copolymeren, Ethylenvinylacetat-Copolymeren,
Coextrudieren des ersten, zweiten und optional dritten Beschickungsmaterials zu einer Mehrschichtfolie, wobei das erste Material eine erste Schicht bereitstellt, das zweite Material eine zweite Schicht bereitstellt und das optionale dritte Material eine optionale dritte Schicht bereitstellt, die zwischen der ersten und zweiten Schicht angeordnet ist.

## Revendications

1. Matériau en feuille antiadhésif souple comprenant un film multicouche co-extrudé incluant une première couche comprenant une polyoléfine, et une deuxième couche comprenant un matériau à base de silicone choisi parmi des copolymères polydiméthylsiloxane-urée (PDMS-urée), et de 10 % à 50 % en poids, de préférence de 10 % à 40 % en poids, plus préférablement de 15 % à 35 % en poids, le plus préférablement de 20 % à 25 % en poids, d'un composé de compatibilité choisi parmi des terpolymères éthylène-ester acrylique-anhydride maléique, des copolymères éthylène-acide acrylique, et des copolymères éthylène-acétate de vinyle.

2. Matériau en feuille antiadhésif souple selon la revendication 1, dans lequel ladite première couche comprend en outre de 10 % à 50 % en poids, de préférence de 10 % à 40 % en poids, plus préférablement de 15 % à 35 % en poids, le plus préférablement de 20 % à 25 % en poids, d'un composé de compatibilité choisi parmi des terpolymères éthylène-ester acrylique-anhydride maléique, des copolymères éthylène-acide acrylique, des copolymères éthylène-acétate de vinyle.

3. Matériau en feuille antiadhésif souple selon l'une quelconque des revendications précédentes, dans lequel ledit film multicouche comprend en outre une troisième couche comprise entre lesdites au moins première et deuxième couches, ladite troisième couche étant constituée d'un composé de compatibilité choisi parmi des terpolymères éthylène-ester acrylique-anhydride maléique, des copolymères éthylène-acide acrylique, des copolymères éthylène-acétate de vinyle.

4. Matériau en feuille antiadhésif souple selon l'une quelconque des revendications précédentes, dans lequel ledit composé de compatibilité est un terpolymère éthylène-ester acrylique-anhydride maléique.

5. Matériau en feuille antiadhésif souple selon l'une quelconque des revendications précédentes, dans lequel ladite polyoléfine est du polyéthylène.

6. Matériau en feuille antiadhésif souple selon l'une quelconque des revendications précédentes, dans lequel ladite première couche a une épaisseur de 5 µm à 60 µm, de préférence de 15 µm à 40 µm, plus préférablement de 20 µm à 40 µm.

7. Matériau en feuille antiadhésif souple selon l'une quelconque des revendications précédentes, dans lequel ladite deuxième couche a une épaisseur de 0,2 µm à 5 µm, de préférence de 0,5 µm à 2 µm.

8. Matériau en feuille antiadhésif souple selon la revendication 3, dans lequel ladite troisième couche a une épaisseur de 1 µm à 10 µm, de préférence de 4 µm à 8 µm.

9. Matériau en feuille antiadhésif souple selon l'une quelconque des revendications précédentes, où ledit matériau en feuille antiadhésif souple a une épaisseur de 8 µm à 80 µm, de préférence de 10 µm à 40 µm, plus préférablement de 15 µm à 30 µm.

10. Emballage libérable pour un article absorbant conditionné individuellement, ledit emballage libérable comprenant un matériau en feuille antiadhésif souple selon l'une quelconque des revendications précédentes.

11. Article absorbant conditionné individuellement comprenant un article absorbant ayant un côté faisant face au corps, un côté faisant face au vêtement, deux côtés longitudinaux et deux extrémités transversales, ledit article absorbant ayant un élément adhésif sur ledit côté faisant face au vêtement, un emballage libérable se trouvant au-dessus dudit côté faisant face au vêtement dudit article et fixé de manière libérable audit élément adhésif, ledit article absorbant et ledit emballage étant plié en tant qu'une unité autour d'au moins un axe de pliage pour définir un conditionnement comprenant ledit article absorbant, dans lequel ledit emballage libérable est selon la revendication 10.

12. Procédé de fabrication d'un matériau en feuille antiadhésif souple selon l'une quelconque des revendications 1 à 9, comprenant les étapes consistant à :
fournir une première alimentation comprenant une polyoléfine et, facultativement, de 10 % à 50 % en poids d'un composé de compatibilité choisi parmi des terpolymères éthylène-ester acrylique-anhydride maléique, des copolymères éthylène-acide acrylique, des copolymères éthylène-acétate de vinyle,
fournir une deuxième alimentation comprenant un matériau à base de silicone choisi parmi des copolymères polydiméthylsiloxane-urée et de 10 % à 50 % en poids, de préférence de 10 % à 40 % en poids, plus préférablement de 15 % à 35 % en poids, le plus préférablement de 20 % à 25 % en poids, d'un composé de compatibilité choisi parmi des terpolymères éthylène-ester acrylique-anhydride maléique, des copolymères éthylène-acide acrylique, des copolymères éthylène-acétate de vinyle,
fournir facultativement une troisième alimentation constituée d'un composé de compatibilité choisi parmi des terpolymères éthylène-ester acrylique-anhydride maléique, des copolymères éthylène-acide acrylique, des copolymères éthylène-acétate de vinyle,
co-extruder lesdites première, deuxième et facultativement troisième alimentations en un film multicouche, dans lequel ledit premier matériau fournit une première couche, ledit deuxième matériau fournit une deuxième couche et ledit troisième matériau facultatif fournit une troisième couche facultative comprise entre lesdites première et deuxième couches.
